# EUROPEAN PATENT APPLICATION

(11) **EP 4 114 042 A1**
(43) Date of publication of application: **04.01.2023**
(21) Application number: 21182398.4
(22) Date of filing: 29.06.2021
(51) Int. Cl.: H04S 7/00, A61B 5/055

(54) **IMMERSIVE AUDIO IN MEDICAL IMAGING AND INTERVENTIONS**

(71) Applicant: Koninklijke Philips N.V., 5656 AG Eindhoven (NL)
(72) Inventor: LEUSSLER, Christoph Günther, 5656 AE Eindhoven (NL); AMTHOR, Thomas Erik, 5656 AE Eindhoven (NL)
(74) Representative: Philips Intellectual Property & Standards

(57) **Abstract**

The present invention relates to a method (100) for providing audio to a subject (200) undergoing a diagnostic imaging or medical procedure. The method comprises receiving (101) at least one audio stream and receiving (102) a definition of at least one source point in a three-dimensional space. The method comprises transforming (103) the at least one audio stream into at least two audio signals for corresponding sound outlets, in which these sound outlets are in a predetermined physical spatial configuration with respect to the subject during the procedure. The method also comprises outputting (105) the at least two audio signals to the corresponding sound outlets to produce sound audible by the subject. The step of transforming (103) comprises spatially encoding the audio stream into the at least two audio signals such that the produced sound corresponding to the audio stream appears, to the subject, to originate from the defined source point.

## Description

### FIELD OF THE INVENTION

The present invention relates to the field of medical devices, such as medical imaging devices, e.g. computed tomography (CT) and magnetic resonance imaging (MRI) scanners. More specifically, the invention relates to a method, device, system and computer program product for providing an immersive audio experience to a subject undergoing a diagnostic imaging examination or medical procedure.

### BACKGROUND OF THE INVENTION

In diagnostic imaging, a patient, or generally a subject, may need to be placed in a dedicated environment for acquiring imaging data. This includes systems for conventional radiological imaging, such as computed tomography scanners and magnetic resonance imaging scanners, and nuclear medicine imaging systems, such as positron emission tomography scanners and single photon emission computed tomography scanners. For the purpose of the present disclosure, this may also include imaging devices that are combined with therapy and/or surgery systems, i.e. that are not solely intended for diagnostic purposes, such as known in radiotherapy, catherization suites, ablation systems and the like.

However, such environment suitable for image acquisition may have a distracting or discomforting effect on the subject undergoing the examination (or procedure). For example, a patient positioned in an MRI bore may feel uncomfortable due to the surrounding cylindrical magnet bore cylinder. Claustrophobia can be triggered by undergoing an MRI or CT scan, and/or other features of undergoing the examination or procedure, such as being fixated by a radiation therapy mask. In most diagnostic and/or therapeutical settings, at least when the patient is not unconscious, e.g. for autonomous imaging, it may be essential or strongly preferred that the patient is relaxed and does not panic during the session.

For example, a magnetic resonance imaging examination comprises a diagnostic test that requires a patient to lie still for prolonged periods of time within a claustrophobic and noisy environment. This can be particularly difficult for children to tolerate. Furthermore, it is not uncommon for psychiatric patients, or patients with a neurological condition, to require such examination for accurate diagnosis, while such individuals may also have a lower tolerance for such discomforting environment. Often, general anesthetics (GA) is required to be able to perform the procedure, which comes at a considerable cost and a risk in terms of patient safety.

Under normal conditions, e.g. in everyday life, the brain receives auditory signals that encode spatially separated sounds, e.g. when not wearing a headset. However, during an MRI scan, and/or other medical diagnostic and/or therapeutical procedures, a patient may be provided with an active acoustic headsets or earplugs. Furthermore, instructions from an operator or from other medical personnel may be delivered via an audio interface, e.g. connected to such headset or to an intercom system. The use of a mono-acoustic audio source (e.g. an intercom in the MRI bore) may create an oppressive, for patients prone to phobia, anxiety, depression, psychosis or other mental conditions possibly even nightmarish feeling for the patient. Such impression may be caused by the failure of the subject's brain to localize the direction of the sound. This may be even further enhanced by poor audio conduction properties of the audio system of the environment, such as static noise and/or a limited or patchy frequency bandwidth.

Virtual reality (VR) is an emerging technology that can be implemented at a relatively low cost within a healthcare setting. This can, advantageously, be used to reduce fear and discomfort associated with various anxiety-inducing situations and medical procedures.

Using sound processing techniques that manipulate the sound produced by stereo speakers, surround-sound speakers, speaker-arrays, and/or headphones; three-dimensional (3D) audio effects can be obtained. This allows sound sources to be, virtually, placed substantially anywhere in 3D space, e.g. strongly independent from the positions of the actual speakers used in the real environment. For example, a virtual sound source may be placed behind, above or below the listener, e.g. even if the speakers (e.g. in headphones) are essentially laterally located to the subject's ears.

For example, 3D audio processing may use the spatial domain convolution of sound waves using a so-called head-related transfer function (HRTF). Thus, sound waves (in their signal and/or digital representation) may be transformed using the HRTF or filters associated therewith, possibly in combination with other techniques, e.g. for cross-talk cancellation, to mimic natural soundwaves which emanate from one or more specific points in a virtual 3D space. This may also be combined with methods to simulate a specific sound environment, e.g. by simulating echo, frequency transmission, reverberation and/or similar audio characteristics of a chosen environment. By using such audio processing techniques, the brain can be tricked to interpret the auditory signals received from the ears via the auditory nerves to construct a mental spatial representation that places different sounds in different 3D locations, even though the sounds may just be produced from only two speakers, and/or, generally, by a set of speakers at real positions in space that can be substantially different from the virtual locations associated by the brain with the perceived sounds.

US 5,877,732 provides an example of a three-dimensional high-resolution video and audio system that is suitable for use within the magnetic field of an MRI scanner bore. Video images are provided to a patient via a relay lens system, and corresponding sound is delivered via a non-magnetic path. A non-magnetic shielded video source can be used in an MRI environment, in which the generated video image can be transmitted via an optical mechanism, e.g. a lens system, to an optical receiver (e.g. to goggles, a headset or a head-coil integrated structure). Likewise, non-magnetic shielded audio speakers, e.g. shielded transducers, can be used to generate audible sound, which can be transmitted to a noise-canceling headset via a suitable non-magnetic path.

### SUMMARY OF THE INVENTION

It is an object of embodiments of the present invention to provide an immersive experience to a subject during a diagnostic imaging and/or medical intervention, such as during radiological or nuclear medicine imaging sessions, radiotherapy, surgery (without anesthesia), stone ablation, and/or other medical procedures.

It is an advantage of embodiments of the present invention that an illusion of a natural space, e.g. a large space, can be created for the subject even when situated in an unfamiliar, discomforting and/or constricted space during the procedure.

It is an advantage of embodiments of the present invention that embodiments may be particularly suitable for improving the comfort of a subject during a magnetic resonance imaging session, and/or during a computed tomography imaging session.

It is an advantage of embodiments of the present invention that a good compatibility with common MRI and/or CT systems can be achieved. For example, a good integration with an MRI system can be achieved, e.g. using an in-bore audio system.

It is an advantage of embodiments of the present invention that the auditory impression of a large space can be created for a patient when positioned in an MRI scanner bore, which is typically relatively small and narrow. Therefore, feelings of anxiety and/or claustrophobia may be reduced and/or overcome, and/or patient experience can be improved. With sounds (e.g. voices, music, soothing sounds, ...) seemingly originating from specific positions in space, e.g. from virtual positions at a considerable distance, feelings associated with being confined in a scanner bore and/or wearing headphones can be reduced. Likewise, a subject can be distracted from the operation of a CT gantry, which may appear, at least to some patients, as unnatural, fear-inspiring and/or trigger negative associations.

It is an advantage of embodiments of the present invention that the patient experience can be further enhanced, e.g. negative associations and/or emotions can be reduced, by using two-dimensional or three-dimensional video technology.

It is an advantage of embodiments of the present invention that an operator can control the spatial properties of the auditory (and/or visual) virtual experience to a subject under study, e.g. such as to select a suitable environment, tune parameters to optimize the experience, and/or to empirically search for a more comfortable setting for the subject.

It is an advantage of embodiments of the present invention that an operator can provide information and/or instructions to the subject being examined in way that is comfortable to the subject. Furthermore, such communication system can advantageously be combined with the advantages in terms of patient comfort provided by the present invention.

It is an advantage of embodiments of the present invention that a pre-existing system component can be easily modified, upgraded or reconfigured in a cost-efficient manner.

A system, device, method and computer-program product in accordance with embodiments of the present invention achieves the above objective.

In a first aspect, the present invention relates to a method for providing audio to a subject undergoing a diagnostic imaging or medical procedure. The method comprises receiving at least one audio stream, and receiving a definition of at least one source point in a three-dimensional space. The method comprises transforming the at least one audio stream into at least two audio signals for corresponding sound outlets, the sound outlets being in a predetermined physical spatial configuration with respect to the subject during the procedure. The method also comprises outputting the at least two audio signals to the corresponding sound outlets to produce sound audible by the subject. The step of transforming comprises spatially encoding the audio stream into the at least two audio signals such that the produced sound corresponding to the audio stream appears, to the subject, to originate from the defined source point.

In a method in accordance with embodiments of the present invention, the step of transforming may comprise applying different filters to the audio stream to obtain the corresponding at least two audio signals, in which each of said filters is constructed by taking a head-related transfer function into account that represents a response needed at the corresponding sound outlet to emulate sound emitted from the source point. The filter may be implemented explicitly or implicitly, e.g. by a trained machine-learning model.

A method in accordance with embodiments of the present invention may comprise a calibration before performing the procedure, in which a response recorded by a microphone inserted in the ear canal of the subject to an emitted test sound is used to calibrate said head-related transfer function, e.g. a separate HRTF for each ear, e.g. in which the test sound or sounds include a wide range of test frequencies of sound (e.g. a broadband sound or combination of a plurality of test frequencies in sequence and/or in superposition) and/or are emitted from a plurality of sample points in space to determine a frequency response at the ear drum as function of source location in space of the test sound.

A method in accordance with embodiments of the present invention may comprise receiving position and/or orientation information from a diagnostic imaging or medical system adapted to perform said imaging or medical procedure. The position and/or orientation information may be indicative of a position and/or orientation of the subject and/or the subject's head (and/or the sound outlets, e.g. headphones in fixed spatial configuration with respect to the subject). The step of transforming the at least one audio stream may comprise taking said position and/or orientation information into account, such that a change in said position and/or orientation is compensated for, e.g. by adjusting the at least one source point and/or a representation of the predetermined spatial configuration of the sound outlets, and such that a virtual sound space is simulated by the transformation that is independent of changes of the spatial configuration of the sound outlets and/or position and/or orientation of the subject during the procedure.

In a method in accordance with embodiments of the present invention, the receiving of position and/or orientation information may comprise receiving a position and/or orientation from a controller of an automated patient couch that supports the subject during the procedure.

In a method in accordance with embodiments of the present invention, the receiving of position and/or orientation information may comprise receiving pose information indicative of a pose of the subject during the procedure.

In a method in accordance with embodiments of the present invention, the receiving of position and/or orientation information may comprise receiving sensor data, in which the subject is monitored during the procedure by acquiring said sensor data, wherein said sensor data is furthermore analyzed to determine a position and/or orientation of the subject and/or the subject's head.

In a method in accordance with embodiments of the present invention, the sensor data may comprise video camera images in the visible and/or infrared wavelength range, and/or light detection and ranging (LIDAR) data, and/or radio detection and ranging (RADAR) data.

In a method in accordance with embodiments of the present invention, the sound outlets may comprise at least a left earpiece and a right earpiece of a headphone set, in which said predetermined spatial configuration corresponds to the subject wearing said headphones during the procedure.

In a method in accordance with embodiments of the present invention, the at least one audio stream may comprise a digital file or data structure encoding audio data that was recorded or generated in advance.

In a method in accordance with embodiments of the present invention, the at least one audio stream may comprise a digital or analog signal from a microphone or sound recorder such that the signal represents live-recorded sounds.

In a method in accordance with embodiments of the present invention, the at least one audio stream may comprise an audio stream in mono audio format, in stereo audio format, in a surround sound format and/or in a format that spatially encodes audio.

In a method in accordance with embodiments of the present invention, receiving the definition of one or more source points may comprise presenting a graphical user interface to an operator in which the one or more source points are represented graphically, in which the graphical user interface allows the graphical representation of the at least one source point to be manipulated by the operator so as to define and/or change said at least one source point.

A method in accordance with embodiments of the present invention may comprise outputting a two-dimensional or three-dimensional video stream to at least one display to show visual content to the subject to form a (e.g. visual and auditory, e.g. consistent in sound and image) virtual or augmented reality simulation in combination with the at least two audio signals.

In a method in accordance with embodiments of the present invention, the at least one source point may be shifted in the three-dimensional space (e.g. along a linear or curved path) during the procedure in accordance with a progress of the procedure so as to represent said progress by perceptual movement of the virtual source of the audio stream for the subject.

In a second aspect, the present invention relates to a device for providing (e.g. binaural) audio to a subject while undergoing a diagnostic imaging or medical procedure. The device comprises an input for receiving at least one audio stream. The device comprises a command center interface for interactively receiving a definition of at least one source point in a three-dimensional space from an operator. The device comprises a three-dimensional audio processor for transforming the at least one audio stream into at least two audio signals for outputting by corresponding sound outlets, in which the sound outlets are in a predetermined spatial configuration with respect to the subject during the procedure. The device comprises an output for outputting the at least two audio signals to said corresponding sound outlets to produce sound audible by the subject during said procedure. The three-dimensional audio processor is adapted to spatially encode the audio stream into the at least two audio signals such that the produced sound corresponding to the or each audio stream appears, to the subject, to originate from its corresponding defined source point or source points.

A device in accordance with embodiments of the present invention mat comprise further input for receiving position and/or orientation information from a diagnostic imaging or medical system adapted to perform said diagnostic imaging or medical procedure, in which the position and/or orientation information is indicative of a position and/or orientation of the subject and/or of the subject's head (and/or of the sound outlets in a fixed arrangement with respect to the subject's head). The three-dimensional audio processor may be adapted to take the position and/or orientation information into account, such that a change in said position and/or orientation is compensated for, e.g. by adjusting the at least one source point and/or a representation of the predetermined spatial configuration of the sound outlets, and such that a virtual sound space is simulated by the three-dimensional audio processor that is independent of changes of the spatial configuration of the sound outlets and/or position and/or orientation of the subject during the procedure.

In a device in accordance with embodiments of the present invention, the input may comprise an intercom microphone for transducing the voice of an operator into the audio stream such that the operator can provide instructions and/or information to the subject by voice.

In a device in accordance with embodiments of the present invention, the input may comprise an entertainment audio source for reading or receiving a music track, an audio track associated with a video, a soundscape file and/or an audio file as said audio stream(s).

In a device in accordance with embodiments of the present invention, the input may comprise an attendant microphone for transducing the voice of an attendant of the subject into the audio stream to comfort (e.g. calm) and/or (e.g. emotionally) support the subject during the procedure.

A device in accordance with embodiments of the present invention may comprise a web application or native application for executing on a smartphone to operate said smartphone as a remote microphone. The input may thus be adapted to connect to said smartphone application for remotely receiving the attendant microphone input.

A device in accordance with embodiments of the present invention may comprise headphones that comprise at least two sound outlets for emitting sound to the respective ears of the subject.

A device in accordance with embodiments of the present invention may comprise a video display configured to be viewed by the subject during the procedure. For example, the device may comprise a graphics processor to create video content for the video display, e.g. 2D or 3D video content (e.g. stereoscopic content to be provided to each eye via the video display to create the visual perception of depth), in which the video content may be coordinated with the at least two audio signals to create a consistent virtual reality or augmented reality experience.

In a third aspect, the present invention relates to a diagnostic imaging system. The system comprises a diagnostic imaging scanner, e.g. a CT scanner or MRI scanner, for acquiring diagnostic images of a subject while the subject is positioned inside an examination zone of the scanner. The system also comprises a device in accordance with embodiments of the second aspect of the present invention.

Additionally, a further aspect of the present invention relates to a medical treatment system for performing a medical procedure, e.g. a radiotherapy system, comprising the device in accordance with embodiments of the second aspect of the present invention. Optional features of a diagnostic imaging system in accordance with embodiments may equally relate to optional features of the medical treatment system in accordance with embodiments, mutatis mutandis.

A diagnostic imaging system in accordance with embodiments of the present invention may comprise an automated patient couch for supporting the subject in the examination zone during the image acquisition, wherein position and/or orientation information of the automated patient couch is provided via said further input to the device in accordance with embodiments of the second aspect of the present invention.

In a fourth aspect, the present invention also relates to a computer-program product for performing a method in accordance with embodiments of the first aspect of the present invention when executed by a computer or other suitable processor.

The independent and dependent claims describe specific and preferred features of the invention. Features of the dependent claims can be combined with features of the independent claims and with features of other dependent claims as deemed appropriate, and not necessarily only as explicitly stated in the claims.

### BRIEF DESCRIPTION OF THE DRAWINGS

Fig. 1 shows a method in accordance with embodiments of the present invention.
Fig. 2 illustrates an example of a usage case (or, in other words, of an area of application) for a method in accordance with embodiments of the present invention.
Fig. 3 illustrates aspects of a method and device in accordance with embodiments of the present invention.
Fig. 4 shows, schematically, a user interface example for controlling a sound environment, as applicable in a method and/or device in accordance with embodiments of the present invention.
Fig. 5 shows, schematically, another user interface example for controlling a sound environment, as applicable in a method and/or device in accordance with embodiments of the present invention.
The drawings are schematic and not limiting. Elements in the drawings are not necessarily represented on scale. The present invention is not necessarily limited to the specific embodiments of the present invention as shown in the drawings.

### DETAILED DESCRIPTION OF EMBODIMENTS

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

In a first aspect, the present invention relates to a method for providing audio to a subject undergoing a diagnostic imaging or medical procedure, e.g. while positioned in a magnetic resonance imaging system, computed tomography system, radiotherapy system, and the like.

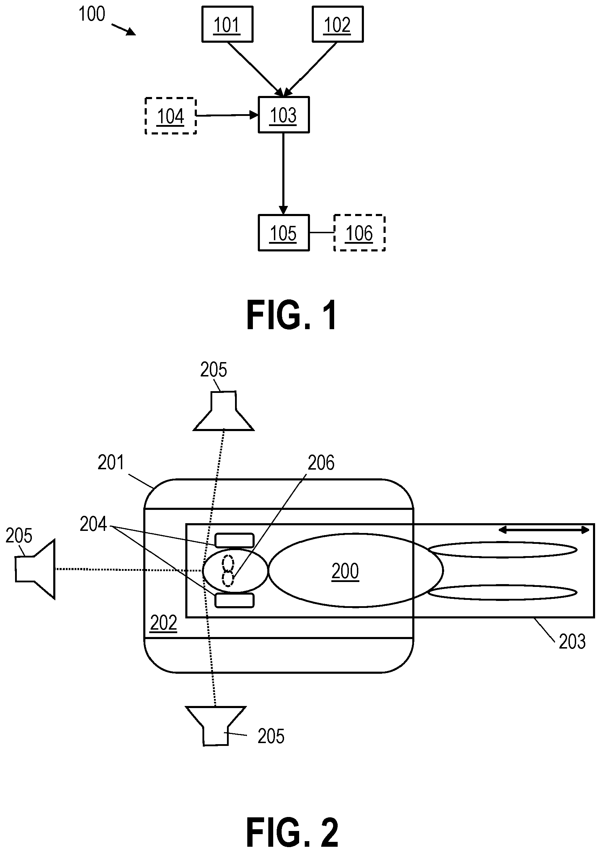

Referring to Fig. 1, an illustrative method 100 in accordance with embodiments of the present invention is shown. The method may provide, for example, an in-bore three-dimensional (3D) audio system for e.g. MRI or CT, which can advantageously create the illusion to a patient of a familiar, natural and/or comforting space (e.g. a large space, such as an outdoor space). This can reduce the risk for claustrophobic reactions and/or anxiety, and may improve, generally, the patient experience. The audio system can furthermore be combined with two-dimensional (2D) or 3D in-bore video technology, as is discussed further hereinbelow, such that this patient experience and/or calming effect can be further enhanced by visual content.

A conceptual representation of an illustrative usage context for a method in accordance with embodiments of the present invention is shown in Fig. 2. In this example, a subject 200, e.g. a patient, is positioned inside an examination zone of an MRI scanner 201, e.g. inside the inner bore volume 202 that is defined (e.g., in other words, enclosed or surrounded) by the MRI bore. The subject 200 may be placed on a patient couch 203, which may be (e.g. is typically or commonly) automated and controllable by the MRI system and/or an operator. As explained further hereinbelow, position information that defines the position of the automated patient couch can (optionally) be used to, advantageously, create a (e.g. even more) realistic or consistent audio (and/or visual) experience for the subject, e.g. particularly to keep the experience consistent when the subject is repositioned in actual space.

In accordance with embodiments, the subject 200 may be provided with headphones 204, or may be able to hear sounds emitted by at least two different sound outlets of an audio system, i.e. sound sources such as speakers. The positions in space of the at least two different sound outlets is sufficiently different such that they can produce sound that reach the subject's brain differently, i.e. such that spatial information can be inferred by the subject's brain from slight differences between the auditory signals reaching the left and right ear, e.g. small phase differences. Clearly, headphones may be particularly suitable for delivering a (stereo) audio signal to the subject, and may also reduce the level of ambient noise perceivable by the subject, e.g. loud noises typically associated with operating an MRI scanner. However, embodiments are not necessarily limited to headphones alone, e.g. may rely on a (at least stereo) speaker system in the vicinity of the subject, e.g. in the imaging or intervention room where the procedure is being carried out, e.g. integrated in a magnet bore cover of an MRI system or attached thereto.

In accordance with embodiments of the present invention, the audio provided to the subject 200, e.g. via headphones 204, can be processed such that (an) audio stream(s) appear to originate from one or more (virtual) positions 205 in space (i.e. which "virtual" positions can be unrelated to or independent of the actual position of the sound outlets, e.g. the positions of the earpieces of the headphones 204). Thus, one or more different virtual sound sources can be configured, such that, during the examination, information and/or instructions from an operator (e.g. a nurse) and/or entertainment sounds can be played as seemingly originating from this/these virtual sound source(s), e.g. from the back, the sides, above, or below.

Optionally, the subject 200 may also be provided with a video display 206, e.g. a pair of 3D video goggles. The video display may allow the subject to view an entertainment feed (which may be coordinated or consistent with the auditory experience provided via the headphones 204), view information, receive (visual) instructions and/or evoke brain potentials (directly or via a task to be performed on the basis of the displayed content), e.g. for functional brain imaging. The video display may be essentially two-dimensional, e.g. a projection screen or conventional display may be viewed (optionally via a mirror, e.g. in case where a direct line of view may be difficult, e.g. in MRI or CT imaging). The video display may also be three-dimensional (3D) or "2.5D", e.g. may provide stereo video content by providing different video content to each eye. For example, the subject may be fitted with a pair of display goggles, in which video content is delivered to each eye separately (e.g. via small integrated-circuit displays, e.g. LED, LCD, TFT, OLED, QLED, ... technology, and/or via laser, small-scale integrated optics and/or optic fiber technology; without limitation to any of the mentioned illustrative technologies).

Referring back to Fig. 1, the method 100 comprises receiving 101 an audio stream (e.g. an audio signal, a digital file or data structure encoding a time series of audio data, and/or another form of audio representation). The audio stream may be received from a storage device, e.g. a hard disk, a network interface to a storage system, a (e.g. persistent or non-persistent) digital memory, a compact disc or similar storage medium, a flash memory, and/or other digital data storage means as known in the art. The audio stream may also be received from a microphone or other sound recording device. The audio stream may be received in digital form, or in another form, such as an optical signal, an analog (electric) signal, etc. The audio stream, e.g. when not already received in digital form, may be digitized, e.g. using an analog to digital converter (ADC). Multiple audio streams may be received and combined, e.g. using an analog or digital audio mixer, or obtained as input for further steps of the method as separate streams to be processed (e.g. synthetically combined in a virtual space environment as discussed hereinbelow).

The method comprises receiving 102 a definition of one or more spatial coordinates (e.g. 3D or 4D coordinates, e.g. 3D space coordinates in combination with a time offset) in a (e.g. virtual) three-dimensional space. Thus, positions in a space are defined for the or each audio stream to project the audio stream from that position, at least perceptually by spatial encoding.

For example, the spatial coordinate may be received as digital input from a storage device (e.g. as discussed above, but not necessarily the same storage device as used for the audio stream), and/or received from an operator via a user interface, e.g. a graphical user interface that allows a point in space to be entered (e.g. via input fields for a vector of values representing the point, by selecting the point in a representation of the space, by moving a cursor or marker on a map or other spatial representation, etc.). The user interface may be configured to allow a direct definition of the point in space by an operator, or in an implicit manner, e.g. selecting from a list of possible scenarios, patient characterizations, imaging or therapy procedures, defining another parameter from which a suitable point can be determined (e.g. rating an anxiety level of the subject undergoing the procedure), another alternative for implicit definition of the point and/or combinations thereof. Thus, a command center application may be provided that allows the operator to control the positions of the virtual sound sources in space, e.g. to control the three-dimensional origins of sounds, music, and voice interaction. Such application may also allow further parameters to be selected, e.g. a configuration of a headphone or speaker system. For example, the application may provide a calibration or configuration routine to select a position of sound outlets (e.g. speakers or headphones), to calibrate a distance between sound outlets, to set a position and/or orientation of the subject's head during the procedure (and/or receive such information from a system used during the procedure, e.g. a position of an automated patient couch).

It is noted that the step of receiving 102 the definition may include receiving a plurality of spatial coordinates, possibly even substantially large number. As one example, an operator may provide a source point for an ambient sound, or multiple source points for multiple ambient sounds (e.g. recording sounds of nature or other familiar environment, such as city sounds, ocean waves, desert sounds, jungle sounds, ..., and/or a source point for a live audio input (or multiple source points/live inputs), e.g. from a microphone used by the operator to provide instructions and/or information or to interact with the subject. As already noted, such definition of the source(s) may be implicit, for example, such that a number of source points can be conveniently defined by a simple selection of one or a few points, a distance (e.g. a radius of a sphere on which multiple sources are dispersed), a scenario selection, (an)other parameter(s) that can be used to infer suitable source points for multiple audio streams, and/or other alternatives.

The received audio streams or audio streams may be in mono-audio format, stereo audio format and/or more fully spatially encoded, e.g. as surround sound or another known alternative to encode spatial audio. Surround sound may refer to various different types or formats of surround sound encoding, which are generally to be considered as covered by embodiments of the present invention, e.g. quadrophonic, "5+1", "7+1", and many other formats (the "+1" channel traditionally refers to a separate channel for low sounds, i.e. a subwoofer channel). Furthermore, regardless of the number of channels (e.g. mono/1, stereo/2, 4, 5, 5+1, ...) in a stream, various type of compression, encryption and/or error correction can be used, as is well-known in the art. For example, an audio stream may be uncompressed and at a specific sampling rate and bit depth (e.g. 44.1kHz, 48kHz, ... and 12 bits, 16 bits, 24 bits, 36 bits, .., big-endian or little-endian, ...), or may be compressed using a lossless or lossy compression technique, e.g. using pulse-code modulation (PCM), dynamic range compression, MPEG Layer III (MP3) compression and many more known options for compression technology. It is specifically noted that formats to spatially encode audio, other than conventional surround audio techniques, are not necessarily excluded. Examples may include wave field synthesis (WFS) representations, ambisonics (e.g. also referred to as full-sphere surround sound) and related techniques/formats (e.g. specifically, formats aiming to represent multi-source or otherwise spatially annotated audio in a way that deviates from the more conventional representation by separate channels).

Thus, the definition of the spatial coordinate(s) may be combined with spatial information that is already encoded in the audio stream(s). Alternatively, the audio stream, some of streams or all streams may, however, also be in a mono audio format, e.g. such that the received 102 definition in space may be the only source of spatial information for the audio processing to be applied.

If the (or one/some of the) audio stream(s) already comprises spatial data, e.g. is in a surround audio format or in another format that can encode spatial sound, the sound information in the stream can be transformed (e.g. based on the spatial coordinates received and/or transformations being computed on the basis thereof) such as to, for example, change a spatial source distance of one or more components that already have a direction encoded, and/or changing an orientation plane of the spatial encoding of the audio. For example, the orientation of a patient wearing headphones may be taken into account to ensure that a spatial vertical axis of the sound recording (e.g. the surround audio stream) is in agreement with an actual vertical direction in the room (e.g. to avoid, for instance, the impression of bird song sounds coming from one side of the room, e.g. superior with respect to the patient's head when lying prone on a couch, whereas the illustrative recording would intend bird song to be originate from above an upright listener, e.g. filling the skies above and around the listener).

Furthermore, where reference is made to a point in space or points in space, it will be understood that this is not necessarily a single point in space, nor necessarily a discrete definition of points in space. For example, the definition may be in the from of a region (volume) in space, or a non-discrete spatial distribution (or a combination of discrete and continuous components).

The method 100 comprises transforming 103 the audio stream or audio streams to a plurality of (i.e. at least two) audio signals corresponding to sound outlets having (different; i.e. at least two) spatial positions in relation to the subject during the examination. These at least two spatial positions correspond to sound outlet positions of a speaker or headphones system via which the sound is provided to the subject. The at least two predetermined spatial positions may be defined in the same space as the one or more spatial coordinates in the received definition 102, i.e. in a virtual space to simulate, or in a real world coordinate system, e.g. of the room where the imaging procedure or intervention is performed. This allows the positions to project the audio streams from to be related to the positions of actual sound outlets, i.e. from which sound is emitted to be perceived by the subject. However, if different reference frames are used, the step of transforming 103 may comprise calculating a coordinate transform between virtual space coordinates (in which the definition 102 is provided) and the sound outlet coordinates (e.g. of a headphones system, e.g. in real room coordinates).

The method 100 may comprise receiving 104 position information from the diagnostic imaging or medical (e.g. therapy) system used for the procedure. This position information may consist of or comprise spatial information or other data from which the sound outlet positions can be deduced (e.g. directly, or in combination with other information) or updated (e.g. allowing a relative move to be determined). For example, the position information may comprise the position of a patient (e.g. of a patient couch, e.g. a control parameter indicative of the current position of an automated couch), pose information (e.g. a selection of the type of procedure in which the patient is positioned in a specific pose), and/or information about the sound outlets, e.g. a selection of auxiliary equipment used, e.g. a type selection of headphones in use. Other data from which the sound outlet positions can be deduced (or which may aid in automatically determining the positions, e.g. in combination with other data) may comprise, for example, camera observation information (e.g. a camera image feed) from which the position of the sound outlets and/or the patient can be determined. For example, an image (feature) recognition algorithm can be used to detect the position of the patient's head, of an MRI head-coil, of headphones, and/or of other salient features in a video feed, from which the current position of the sound outlets can be determined (or such that this video information can be taken into account in combination with other information sources to determine the position).

Other examples, other than video camera monitoring, may include receiving data from other tracking or positioning systems, such as LIDAR or RADAR observation for position sensing, and/or positioning tags on the head and/or headphones (e.g. which may be localized by GPS-like radiofrequency signal interferometry).

The step of transforming 103 may thus take position information received 104 from the system into account, e.g. to update the relative position of virtual sound source points with respect to the sound outlet positions, e.g. to maintain consistency with a simulated space.

Thus, the system, e.g. an MRI or CT system, can provide information on the patient position to be taken into account in the step of transforming 103 the audio stream(s). For example, information from the MRI system about the patient positioning can be fed back into the three-dimensional audio processing algorithm, such that sound origins are tied to the room's coordinate system even for different patient poses (e.g., on side, supine, prone), position (e.g. table offset along axial direction) and angulation of head and headphones.

If a uniform (i.e. the same) spatial reference space is not used, e.g. if all data is not explicitly expressed in world coordinates (i.e. real room coordinates), a further transformation(s) may be used to express room coordinates in virtual space coordinates and/or vice versa, e.g. such that the transformation 103 can act on the audio stream data (if including spatial information), the source point definition (received in step 102) and the sound outlet positions (used in step 103) in a same reference frame. Thus, while for the sake of simplicity, all spatial information can be treated in real world coordinates, it is relatively straightforward to use simple coordinate transformations to process the data in an arbitrary spatial reference frame, e.g. at least in so far in a final step of the transformation the output is brought into a coordinate system that accurately (without limitation on the required accuracy, e.g. 'approximately' may suffice) encodes at least the relative position of the sound outlets with respect to each other, and the relative orientation and/or positions thereof with respect to the point(s) from which the audio stream(s) are to be virtually projected.

Note that for a stereo output, e.g. a stereo headphones system, no information on the spatial positions of the outlets may, strictly, be required, except for a relative scaling of the virtual space in which the received spatial information in step 102 (and optionally the spatially encoded information in the audio stream/s) is described to a unit distance between the headphone outlets, and even the latter may be, in a particularly simple system, be assumed fixed or approximated by an average head width. Nonetheless, preferably, orientation information is also taken into account, e.g. such as to bring a reference horizontal and/or vertical plane of the virtual space and a headphones axis orientation (e.g. dependent on head orientation of the subject) into alignment. Furthermore, it may also be preferred to take position information of the subject into account, e.g. when the subject is translated (or reoriented) during the procedure, e.g. by an automated patient couch, such as to create the impression of movement through the virtual space, in a consistent manner, when the subject is moved (in actual reality). This may be particularly relevant for creating a soothing and/or calming virtual experience, since a discrepancy between sensed motion and a constructed headspace based on auditory (and optionally visual) input could in fact cause a sense of unease and/or alienation, whereas quite the opposite would be intended. The transformation takes the audio stream(s) as input, and transforms and/or combines this (these) input(s) to produce output data that is suitable for output via sound outlets at predetermined positions, e.g. headphones provided to the subject or an at least stereo speaker system audible by the subject. The audio stream is transformed, e.g. by manipulating a phase difference and/or amplitude difference between the different channels (corresponding to the different outlets), or, particularly, manipulating the phase and/or amplitude difference as function of sound frequency (e.g. represented by a transfer function), in such manner that the subject is able to spatially associate the audio stream(s) with (a) perceived point(s) of origin that corresponds to the received 102 definition of the source point(s).

The method 100 comprises outputting 105 the plurality of audio signals.

The audio signals may be in digital form or in an analog form, e.g. after passing through a digital to analog converter (DAC). Thus, the signal may be in the form of digital data that is stored in a memory or streamed to an audio system (e.g. an audio interface, such as integrated in/on a computer motherboard, connected to a computer via an interface, e.g. a universal serial bus interface, or connected to a computer motherboard via an internal interface, e.g. a peripheral component interconnect interface). The signal may be in analog form, e.g. in the form of electrical currents and/or voltages transmitted by conductive wire, an optical signal, pressure waves transmitted through tubes, or other forms of audio signal as known in the art. For example, in magnetic resonance imaging, a headphone system may be used that connects outlets near or in the ears of a subject being imaged via air- or gas-filled tubes to a pressure wave generator that is positioned outside the examination area (e.g. outside the magnet bore) such as not to interfere with the sensitive MRI equipment, e.g. with magnetic fields and/or transduction of radiofrequency signals. In other words, such headphone systems may bear, in a certain sense, some similarity with a conventional stethoscope device (without limitation, e.g. the tubes may be open in/near the ears of the subject as well as form substantially closed systems through which sound waves can propagate while having interfaces in the form of membranes at the sound outlets). Alternatives that conduct sound via a solid pressure wave conductor, e.g. a suitable polymer or metal wire, are not necessarily excluded. Other types of headphone system for use in MRI systems are not necessarily excluded either, e.g. relying on optical and/or electric signals while using proper shielding and interference countermeasures to prevent or substantially reduce undesirable interaction with the image acquisition process.

The method 100 may furthermore comprise outputting 106 a video stream. For example, spatial audio information may be processed in combination with spatial video information and provided in combination to the subject. For example, one or more of the audio streams may have an associated video stream or video streams, e.g. a 2D motion film (or other type of video content) or a 3D motion film (or other video content). Thus, the audio component of this video may be transformed and/or mixed with other audio sources as discussed hereinabove, and the transformed audio may be provided to the subject in combination with the video content, e.g. for entertainment purposes or to provide a soothing or relaxing environment.

The video stream is not necessarily predetermined and/or fixed. For example, the video content (2D or 3D, e.g. in the form of a video component of augmented reality, AR, and/or of virtual reality, VR) may be generated by live processing, e.g. as content of a video game or other virtual environment.

Nonetheless, it is to be noted that, in most circumstances, the options for receiving feedback from the subject to control a game or other dynamic simulated environment during an imaging or medical procedure will be very limited, if not entirely impossible. Likewise, it may not be possible for the subject to move much, e.g. look around, such that, if a 3D video perspective in an AR/VR environment is used, the freedom of this environment might be rather limited, e.g. constrained to a fixed or predetermined field of view in the virtual environment.

However, embodiments comprising such game engine or other interactive content generator are certainly not excluded. In specific applications, this may even be particularly advantageous, despite potential difficulties in receiving control feedback from the subject. For example, for functional magnetic resonance imaging, a game or game-like virtual environment may be used to allow specific brain activity to be detected and imaged that would otherwise be difficult to test. For example, AR/VR techniques may be used in an fMRI paradigm to detect brain activity that is associated with high level brain processing or other aspects of the brain that are difficult to test passively and/or that are strongly linked to spatial perception and/or processing in the brain. Examples may include paradigms to specifically activate memory function, train or test reflexes, (particularly noteworthy) spatial learning and recall, and/or realistic tests (e.g. "real-world scenarios") that are intended to invoke a specific brain response in patients who are not able to perform an alternative complex or abstract task due to impaired memory or cognitive functions.

The audio processing system, or combined video/audio processing system, may also be used to induce or promote a change of state in the subject during the examination or procedure. For example, the subject may be sedated or heavily relaxed during the procedure, and the audio and/or video streams presented to the subject may change throughout the procedure (or toward the end, e.g. shortly before the end) to promote alertness and wakefulness when the procedure concludes, e.g. by increasing intensity of audio/video signals, vividness of colors, changing sound frequency filter characteristics (e.g. filtering lower and passing higher frequencies), including more dynamic and/or more frequent changes in audio and video, and/or other such techniques to promote alertness.

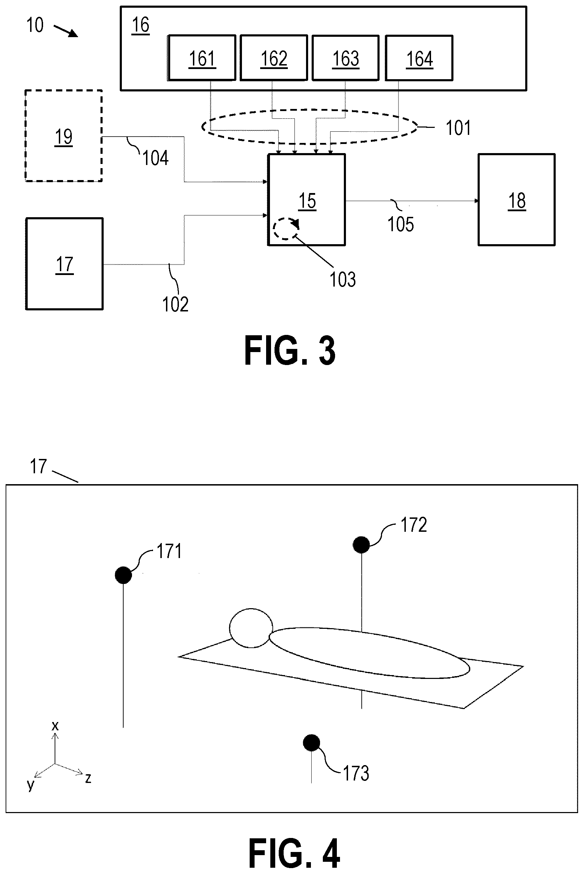

Details of a method 100 in accordance with embodiments of the present invention (and, likewise, also of a device in accordance with embodiments of the present invention) are illustrated by the example diagram in Fig. 3. The method may be performed by a processor, computer or other suitable analog and/or digital processing device. For example, the method may be performed by a 3D audio processor 15 (or, in other words, a spatial audio processor), which may comprise dedicated hardware, e.g. an application specific integrated circuit, specifically designed for carrying out step(s) of the method in accordance with embodiments, or may comprise a software that, in use, executes on a general-purpose (or, at least, programmable and versatile/flexible to some extent) computing and/or signal processing platform to perform steps of the method, or a combination of such software and hardware working together to perform the method.

The method 100 comprises receiving 101 an audio stream as input 16. Thus, the input 16 may be adapted to receive one or more audio streams, e.g. provide an interface with one or more audio sources, e.g. such that the 3D audio processor 15 can access these streams. While the term 'stream(s)' is used, it will be understood that this can refer to live input(s), e.g. to be processed substantially in real-time by the processor 15 (without limitation thereto), or to prerecorded or otherwise earlier-created input(s), or to a combination of both. A prerecorded or otherwise earlier-created input may, for example, comprise a digital file (e.g. accessible, e.g. in its entirety, by the processor, e.g. via a storage device or network interface). For example, an audio stream may, generally and without limitation thereto, provide a (at least one) timeseries of audio data that can be read and/or processed in its inherent chronological sequence. As is well-known in the art, such timeseries may be encoded and/or compressed with various techniques, e.g. some digital representations may require decoding and/or decompression before being able to access the timeseries of audio data. Thus, embodiments of the present invention may also be adapted to decode and/or decompress the input(s), if needed.

Examples of audio sources (or audio streams) may include, a patient/operator intercom system 161, e.g. a microphone or similar recording device for transducing the voice of an operator into an audio signal. One or more further microphones 162 (or similar recording devices) may be used to record additional (e.g. live) sounds, e.g. voices from other people (e.g. a doctor, a further nurse, and/or a family member of the subject), music (e.g. providing a live feed from a musical instrument) and/or ambient sounds (e.g. live recording sounds in a garden, a cafeteria, a waiting room where the subject has arrived from, ...). Another type of audio source may be an entertainment audio source 163, e.g. a music file or playlist, the audio track(s) of a movie (e.g. the movie being played via a visual presentation system to the subject), ambient audio recordings (e.g. soothing sounds and/or "soundscapes"), etc. Clearly, the examples of audio sources provided herein are only illustrative, and can be easily extended with further audio sources 164.

A "soundscape" refers to an acoustic environment to be provided to the human subject that comprises sound recordings (and/or simulated or otherwise artificially created sounds) of natural and/or artificial sound sources in a given environment, e.g. corresponding to a certain archetype of environment. Thus, such combination of sounds can form an immersive environment that evokes an experience in the subject that can be related to associations with and/or memories of the environmental archetype, e.g. such that the sensation of experiencing a particular type of acoustic environment is induced. The sounds may comprise natural sounds, which may include geophonic sounds (e.g. flowing water, ocean waves, wind and/or other weather effects) and/or biophonic sounds (e.g. animal vocalizations), but may also comprise manmade sounds, e.g. anthropophonics sounds such as background voices (often in a muted and/or indistinct form), traffic sounds and/or industrial sounds. The soundscape is not necessarily a true or exact replication of the simulated environment, e.g. may also be conceptualized as a sound performance, e.g. which may include music or other evocative sounds that are not necessarily tied to a specific, real, environment, and/or may idealize or reimagine the basis environment that serves as inspiration. The soundscape may typically comprise a plurality of different sounds, possibly even a very large number of sounds forming an interesting and/or rich ambience, that are spatially dispersed (in a virtual sound space), e.g. such as to create the impression for the listener of being surrounded by, or immersed in, the soundscape. Thus, the soundscape may particularly be provided as a stream (or audio file) in a surround sound format or other format that includes spatial annotation or spatial sound information (particularly, spatial in the sense of allowing sounds to be located by hearing along more than one dimensional axis, e.g. spatially defined to an extent that surpasses the linear balance scale of stereo sound).

The 3D audio processor 15 may be adapted to create audio signals for emission by the sound outlets, e.g. by the patient headphones, so as to be heard by the patient. These audio signals are constructed such as to mimic sound originating from one or more different positions in space, which can be substantially different from the positions of the actual sound outlets, e.g. of the headphones. The audio sources 16 may be connected to the processing system 15, e.g. a user interface may allow one or more audio sources to be selected for feeding into the created audio signals, possibly even in a live configurable manner, e.g. the operator may be able to switch, mute, unmute, select, adjust volume and/or perform another operation on the audio source(s) in substantially real-time (without limitation thereto). As an example, the operator may be able to select an entertainment feed, e.g. a music track, a video (and its associated audio track), a soundscape or other such sound source via a user interface. The operator may be able to activate a microphone such that his/her instructions and/or information can be provided via the operator intercom to the subject.

Another example of an audio source may be a microphone through which a relative or a caretaker of the subject may communicate with the subject. This can be particularly useful for pediatric or elderly patients, and/or patients who are, generally, confused, e.g. in neurological trauma cases or psychiatric care. The use of 3D localization, by the processing 103 (e.g. by an 3D audio processor 15), may be used to assign a spatial (virtual) location to the voice(s) in (apparently) the examination room, such that the patient can identify relatives or other familiar persons (perceptually) nearby. For example, relatives that were accompanying the patient in a preparation or waiting room, prior to being brought to the examination room, can still appear to be standing next to the bed during the examination. This can therefore aid in managing distress and discomfort in, particularly, pediatric imaging and/or for elderly patients.

It is also noted that the microphone (or one/some of the microphones) is not necessarily a wired device, in other words, is not necessarily directly connected by a (e.g. electric signal) wire to the processor 15. For example, a portable microphone or recorder can be used, e.g. which may connect to the audio processor, e.g. directly or indirectly, via a suitable wireless technology, e.g. Bluetooth, Wi-Fi, cellular data network connection (e.g. GPRS, EDGE, 3G, 4G, 5G,..) and/or other wireless communication technologies. Particularly, but without limitation to this example, the portable microphone or recorder can be provided/implemented by/in a mobile telephone, e.g. a smartphone. Thus, a family member or otherwise significant person to the patient can be allowed to connect via a portal to the audio processor, e.g. via a dial-in telephone number, via a web application or via an app for the smartphone, such that sound (e.g. the voice) of the family member (or caretaker, attendant, ...) that is recorded by the (smart)phone can be transmitted to the audio processor, where this audio stream can be processed, e.g. transformed to (virtually) originate from a selected position in space, and accordingly played by the sound outlets (e.g. headphones) to the patient. Optionally, other sounds and/or audio streams may be mixed in into the output sent to the sound outlets, e.g. other sounds or streams may be differently spatially encoded by the processor, such that the patient can more easily identify and distinguish voices of different persons by mentally associating different persons with different locations in space, even if this is not done consciously or actively.

Furthermore, such guest communication app or web application (or dedicated hardware device: e.g. portable microphone, recorder, communicator, patient or client queue tracker, ...) may optionally also be adapted to receive information from the operator, from the 3D audio processor and/or from the medical imaging system (or, where applicable, from the therapeutical or other medical system for managing the procedure that the subject is undergoing). For example, the operator may use a link to the guest app/web application/device to communicate with a caretaker or trusted relative of the imaged subject. This may allow the operator to draw attention to an increase in unrest or anxiety of the subject, such that the family member or caretaker can actively try to calm the subject. A video feed from a camera monitoring the subject may optionally also be relayed to the guest portal (e.g. app, web application, device, ...), e.g. such that the relative(s), caretaker and/or other involved party can view the patient during the procedure. This may advantageously allow a relative or caretaker to observe the patient closely, and actively react by soothing words or sounds, relayed to the patient, when signs of unrest or anxiety start to show.

The operator can interact with this system via, for example, a command center interface 17, e.g. implemented by a graphical user interface. Thus, the audio processor 15 may use information received 102 via the command center interface 17, particularly a definition of one or more spatial coordinates representing a point or points in 3D space from which one or more audio streams received 101 as input 16 are to be, virtually, emitted or projected. As already mentioned, the point(s) in space may also be implicitly defined, e.g. by controlling one or more parameters in the user interface 17 from which the point(s) are determined, and/or a (e.g. continuous) distribution, a surface and/or a volume in space may be defined (directly, e.g. explicitly, or indirectly, e.g. implicitly) rather than merely a point or plurality of points in space (and/or coordinate components thereof).

Fig. 4 shows, conceptually, an example of a user interface for placing (e.g. defining) point sources in three-dimensional space around the subject undergoing the imaging examination (or other medical procedure). In this example, three audio source points are shown, which may, for example, be draggable (in a user interaction) in the represented scene, and/or may be manipulated by the user in another way, e.g. by sliders for controlling parameters, coordinate (numeric) input fields, etc.

For example, a first source point 171 may indicate the position in space from which audio of the patient/operator intercom system 161 is to be projected, e.g. a point in space where the operator's voice (e.g. received as an audio stream from an operator microphone) is intended to seemingly originate from, in the perspective of the subject/patient. Likewise, a second source point 172 may indicate a position in space from which audio of one or more further microphones 162 is to be projected, e.g. a point in space where the voice of an attendant (e.g. a caretaker, a friend or a relative) of the subject is to emanate from in the perspective of the subject, e.g. to provide emotional support. In this example, a third source point 173 is indicated, which may represent a point source from which an entertainment feed, e.g. music, is to be provided, e.g. an audio stream (or streams) from the entertainment audio source 163.

It will be understood that such command center interface may allow other parameters to be controlled as well, e.g. to adjust volume for each of the audio components (e.g. the channels 161,162,163 corresponding to the virtual source points 171, 172, 173), to add (a) further audio source(s) and define corresponding further virtual source point(s), mute and/or unmute audio components, remove channels/source points and/or select a different input source for a source point. The interface may also allow an audio source to be configured, e.g. to select a music track, video, ambient recording (audio and/or video, e.g. a "soundscape") and/or other media file from a list of available content, e.g. using a media manager or play list manager interface.

While a one-on-one correspondence is shown between audio sources and virtual source points, it will also be understood that, in accordance with some embodiments of the present invention, this is not strictly necessary: e.g. multiple source points may be assigned to a same audio source (stream), such that the stream is projected (simultaneously) from a plurality of different (virtual) locations, and/or multiple audio sources may be assigned to a same source point (e.g. mixing signals from different sources before 3D audio encoding the combined stream to the specific source point).

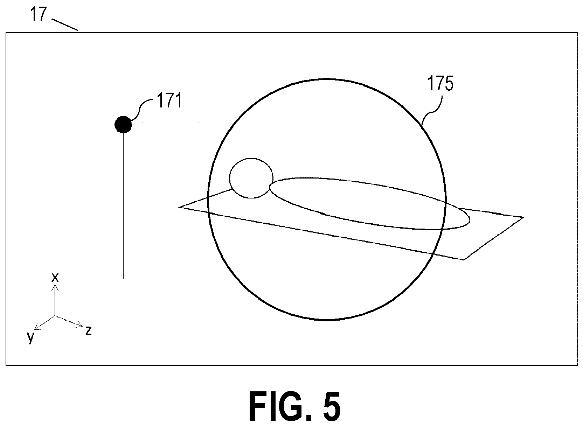

Fig. 5 illustrates a further example of the command center interface 17. In this example, the first virtual source point 171 (e.g. for an operator intercom channel 161) is defined as a point in space in the same manner as in the previous example (FIG 4). However, a 3D surround source 175 is included, which may, for example, represent a source for sound of an entertainment feed, such as 3D audio corresponding to a movie shown to the subject and/or music in surround format. For example, the 3D audio stream may comprise or consist of a plurality (e.g. more than two) sub streams, and/or may encode spatial information. For example, the 3D audio stream may use a surround sound format, e.g. 5.1 surround sound, 7.1 surround sound, Dolby digital, DTS, .... The use of such 3D surround source 175 definition in the user interface is not necessarily limited to entertainment feeds (e.g. 'entertainment' is not to be interpreted in a very strict sense of the word). For example, surround audio may be provided to simulate a relaxing (and/or familiar) environment, such as a 'soundscape', e.g. an ocean or beach soundscape, a jungle soundscape, a desert soundscape, a park soundscape, a outdoor city soundscape, a campfire soundscape, ..., and may, for example, comprise (spatial audio) elements such as birds singing around and/or above the patient, chirping crickets and/or other animal or natural sounds. The representation of a sphere for a surround sound component in the user interface may merely serve to indicate that the surround channel is available or active, or the sphere representation (or alternative form of representation) may have user-controllable properties. For example, the radius of such sphere may be adjustable to represent a uniform scaling transform, e.g. such as to increase/decrease the perceptual distance from which the surround sounds are projected. Likewise, the center of the sphere may be (optionally) draggable to change a perspective, e.g. an offset of the origin of the sound space. Optionally, the orientation of the surround space channel may be adjustable, e.g. to change a horizontal and/or vertical reference plane of the sound channel (even though, for this, a spherical representation of the channel may not be ideal, and/or may need to be extended by some additional markers and/or handles, e.g. of the primary axes or planes). While a single point source 171 and a single surround source 175 are shown in FIG 5, it will be understood that the number of point sources and/or the number of surround sources can vary, in accordance with embodiments of the present invention. For example, different surround sources can be used simultaneously, in accordance with some embodiments, and (e.g.) represented by different spheres in the user interface.

The 3D audio processor 15 may thus be adapted to transform 103 the audio stream or audio streams, received as input 16, to a plurality of (i.e. at least two) audio signals for sound outlets audible by the subject during the procedure. The generated audio signals are provided as an output 18, e.g. transmitted to the sound outlets, e.g. headphones, such that the subject can hear the sound emitted from these sound outlets, e.g. from ear pieces of headphones that (s)he is wearing.

The 3D audio processor 15 may also receive 104 position information from the diagnostic imaging or medical (e.g. therapy) system used for the procedure, e.g. for the MRI or CT scanner system, via a further input 19, e.g. via a network connection, a wireless or wired data link connection, a data bus interface, or the like to allow data to be received from imaging or other medical system for performing the procedure. For example, when the patient couch is moved (e.g. translated) to a different position, the audio processor may receive this information and update the position of the sound outlets with respect to the virtual source points (or equivalently, shift the virtual source points for audio streams in an opposite direction). Thus, the 3D audio processor may be adapted to maintain a consistent virtual audio environment, in which movement of the patient in real space is taken into account to create a realistic impression of a (simulated) sound environment. Thus, a coordinate transformation between the room or bed coordinates to headphone coordinates may be based on information about the patient position taken from the settings or parameters of the MR or CT machine. This may include a patient position defined before the procedure is performed by an operator (e.g. a selection of supine, prone, head-first, feet-first, positioning on the side, etc.), and/or the position of the patient table in the room, e.g. as live feedback or as a control setting defined beforehand. For example, a virtual audio source placed above the patient bed may need to be encoded into different 3D audio signals depending on whether the patient is in supine or prone position. The 3D audio processor may determine such coordinate transformations before creating the 3D audio signals to be supplied to the patient headphones. By taking the position of the patient table into account, an audio source can even appear to be at a stable position in the room while the patient table is moving.

It will be appreciated that embodiments of the present invention may offer additional advantages. For example, a magnetic resonance imaging scanner may be configured to collect functional magnetic resonance imaging data, in which slight changes in the acquired signals can represent a state of blood oxygenation in the brain, and therefore can allow activity in the brain to be visualized and/or localized. Embodiments of the present invention can be used to provide audio queues or triggers from one or more specific directions or (virtual) source locations in space, thus increasing the versatility of possible experimental paradigms that can be implemented. A clear distinction can also be made, i.e. perceptually by the subject, between fMRI paradigm-related queues and triggers, and instructions or information from an operator, e.g. by a different spatial encoding. In other words, the voice of the operator (and/or prerecorded or computer-generated status information or supporting instructions) may be heard from a direction that is clearly distinct from a direction from which paradigm-related events and/or other experimental design related auditive triggers are supplied, e.g. such that the subject can easily and/or instinctively understand whether a sound (e.g. spoken words) are part of the fMRI experimental paradigm or not. This allows the subject to quickly assess whether a sound requires a specific response, e.g. a prescribed action (such as a finger tapping) and/or a prescribed mental processing, in so far of course the experimental design would define such a required response, or should be treated in a more general or natural way, e.g. as conversational information. This perceptual spatial separation between fMRI experimental design audio and other audio sources, not explicitly part of the design, may improve the responsiveness and alertness of the subject in performing the fMRI task. Other potential applications may include psycho/neuro MRI examinations, e.g. to assess dementia, in which the spatial manipulation of sounds can, for example, be used to probe alertness and coherence of (neural) input processes and/or thought processes.

The method may comprise a subject-specific calibration, or may use a generic calibration to relate spatial information to audio signals. In other words, a subject-specific head-related transfer function may be calculated, or a model therefor may be (e.g. implicitly) constructed, e.g. by machine learning. Alternatively, a more generic model (implicit or explicit) may be used, which might be for example less accurate for a specific subject, but more or less able to create a reasonable auditive impression of space (e.g. with a reasonable fidelity of spatial mapping from a technical representation of audio sources in a numerical space to the neural reconstruction of space in the subject based on the audio signals thus created and delivered to the ears) for a wide range of subjects without specific adaptation.

As an example, an individual calibration may be performed in a waiting room or preparation room before the subject enters the examination room or theater for the procedure. For example, microphones built into the headset (headphones) or microphones integrated into patient ear plugs may be used to collect data to generate the head-related transfer function (or an implicit model representative therefor).

In fact, when microphones are inserted inside the ears (e.g. in earplugs), it becomes possible to directly measure the sound that each ear collects (or at least a very good approximation thereof) in response to a specific audio stimulus, e.g. coming from a specific direction with respect to the head axes. Thus, sounds can be emitted from different angles (possibly including both rotation in the horizontal plane, as well as with respect to the vertical axis, e.g. head "yaw" and "pitch" angle variations), e.g. using a calibration system with a plurality of speakers or other sound emitters surrounding the patient's head during the calibration phase (e.g. preferably in preparation or waiting room). Thus, it becomes possible to define the (or each) ear response (e.g. as a transfer function, e.g. a frequency modulation function) as function of origin of the sound.

As an example, it can be assumed that, if the audio signals presented by headphones (or a fixed speaker configuration) as observed at the subject's eardrums are identical to the waveforms that would arrive at the eardrums in a free field as originating from the intended source point(s), the experience of the subject should be the same, i.e. the created signals representative of a virtual space would not be distinguishable from the experience of its real space equivalent. This means that the sound outlets of the headphone earpieces (or other configuration of speakers with sufficient spatial separation) can be provided with processed signals that externalize a sound to its intended virtual source point.

A signal x(t) can be emitted from a predetermined point (e.g. by a speaker at that location) to be received at the eardrum as a signal y(t), which can be measured by a microphone in the ear canal. Likewise, a signal x'(t) can be emitted by a headphone earpiece (or other fixed speaker in space) and will result in a signal y'(t) at the eardrum. Therefore, the experience will be equal if y(t)=y'(t), i.e. the fixed speaker (e.g. from a headphone earpiece) will produce the signal required to simulate the free-field sound from the predetermined point in space. After Fourier transform of the signals in time-domain to express these in frequency domain, represented by the corresponding capitalized symbols X,X',Y,Y', this yields the following expressions: Y=XLFM and Y'=X'HM, in which L is the transfer function of a speaker source in free space (e.g. a test sound emitter), F is the head-related transfer function to be determined, H is the transfer function of headphone (or fixed speaker) to eardrum and M is the transfer function of the microphone picking up the signals in the ear canal. The required equality Y=Y' thus results in X'=XLF/H, such that the desired transfer function LF/H defines a filter to apply to the signal for a predetermined source point to obtain a signal to play via the headphone earpiece (or fixed speaker) to get the same perceptual effect. By sampling y and y' for test sounds x and x', and sampling different test points in space, an array of suitable transfer functions can be obtained, which can be modeled as function of an arbitrary point of origin in space (and/or interpolated). Obviously, this needs to be repeated for each ear piece (or each speaker if a more generic fixed arrangement in space of speakers is used), to get a different processing filter for each component. Other more refined approaches are known in the art as well, e.g. which may split the transfer function to be determined into a phase component (e.g. an interaural time delay) and a magnitude (or "level") component, e.g. using an approximative but more tractable separation of these components of the transfer function.

However, even though the ears of every individual are different, and therefore people have, generally different head-related transfer functions, it will be understood that an approximation or generalization can be constructed that works reasonably well for a wide range of people. Some population specific specialization of the model may further be used to improve the accuracy of such generic model, e.g. using a specific HRTF model for children, adults, other (e.g. more finely defined) age groups, as function of gender, and/or by other population stratification techniques, e.g. based on physical measurements and/or dimensions and/or shape parameters of the ear and/or head.

For example, 3D sounds may be simulated without detailed knowledge of the ear shape (and thus total knowledge of the HRTF), e.g. by simulating an interaural time delay and level differences. The ability of the brain to determine which direction a sound is coming from, may be simulated by presenting a sound first to the ear at the side where the (virtual) sound is coming from and then, with a slight delay and a slight decrease in level, to the other side. The specific delay and level difference may be generated by an explicit model, or by a trained machine-learning model.

For example, Gao and Grauman: "2.5 Visual Sound," published in CVPR 2019 and available via arXiv:1812.04204, presented a deep convolutional neural network that is learnt to decode a monaural (single-channel) soundtrack into its binaural counterpart by injecting visual information about object and scene configurations. Given a video of a scene and mono sound recording, the machine-learning system works out where the sounds are coming from and then distorts the interaural time and level differences to produce that effect for the listener. In this illustrative approach for generating a suitable machine learning model, binaural recordings can be made with a "synthetic ears" construct (to create training data), and the recorded data is annotated with spatial information (in this example, a video clip is recorded from which the direction of different sound components can be inferred).

While the model presented in the article in reference hereinabove is likely not directly applicable to embodiments of the present invention (not excluding embodiments that would rely on this model directly though), it will be understood that a similar machine learning model can be built and trained to create a suitable 3D audio processing model without requiring elaborate changes or an excessive inventive effort.

In an example of a conventional numeric algorithm, it is assumed that a pair of head-related transfer functions, one for each ear of the subject, is known, from a subject-specific calibration approach and/or by constructing a generic model based on empirical (e.g. population-wide) data or detailed modeling of sound propagation in an anatomical model of the human ear (and/or head). This may alternatively be represented in a different form, e.g. as a function defining a common component of both HRTFs and a function describing a difference component between both ear HRTFs, as a numerical approximation (e.g. a table of values, a set of coefficients for a series expansion of the HRTFs, ...), in another representation (e.g. impulse response functions instead of frequency transfer functions, ...). The HRTFs may be subject-specific, e.g. tailored to the subject by a calibration (e.g. using microphone-fitted ear plugs), or may be suitable for a wider population (e.g. for any human; or for a group matched to demographics of the subject, e.g. gender, age, genetic factors, ...). A generic HRTF may be determined based on an idealized head geometry (and/or ear shape/model), and/or as a model based on fitting empirical data.

For example, the HRTF may express a function of spatial coordinates, representative of the origin of a sound with respect to the receiver (e.g. center of the head of the listener), e.g. (polar) coordinates *r*, *θ, ϕ,* or in a far-field approximation the angular coordinates *θ*, *ϕ*. For example, for distances greater that about one meter, the HRTF may generally attenuate inversely with distance (r), such that this parameter can be neglected or at least may not need to be modeled explicitly. Thus, the function can be expressed as *H*(*f,θ,ϕ*), which expresses the ratio of output (heard sound) to input (emitted sound) at a frequency /for a given direction of the sound. For a fixed location (theta and phi), the function H(f) is the Fourier transform of the corresponding head-related impulse response (HRIR).

Many methods are known to simulate a virtual auditory space, taking either a generic model of a HRTF or a more specific ,e.g. calibrated, HRTF into account. For example, the Aureal 3-Dimensional (A3D) and/or Vortex technology of Aureal Semiconductor and/or Creative Labs pioneered to some extent the wide adoption of spatial audio processing and virtual sound environments, e.g. in 3D game applications. This basis has been further extended by Environmental Audio Extensions (1.0, 2.0, 3.0, 4.0, 5.0), which established a convenient framework of software libraries with hardware acceleration for 3D audio environment encoding. The cross-platform audio application programming interface OpenAL provides a further toolset for rendering spatially encoded (3D positional) audio.

A method in accordance with embodiments of the present invention may comprise encoding status information about the procedure (e.g. imaging procedure or medical intervention) in the definition of one or more spatial coordinates of one or more audio source points, e.g. as previously received 102 (e.g. configured by an operator). This allows a sound, e.g. one or more of the audio streams, to change during the procedure to reflect progress through the procedure, e.g. a sound may gradually move away or closer to the subject (at least perceptually) to reflect progress and to allow the subject to estimate a remaining time (or at least give a soft impression of progress). For example, a multi-step procedure, such as an imaging sequence (e.g. collection of a plurality of different images in sequence) may be mirrored, at least conceptually, by shifting a virtual sound origin through a sequence of virtual stepstone locations, e.g. such that the auditory experience can be used as a scan or therapy progress indicator.

Clearly, this can be combined with voice synthesized information to provide more accurate progress information. A voiced status update, e.g. a spoken indicator of progress or remaining time to complete the procedure, can also be used as alternative to a progress indicator based on spatial location of a sound source (as explained hereinabove). Furthermore, both approaches to indicate progress can be combined, as mentioned, e.g. using a soft indication of progress by spatial location of a sound source as well as an explicit mention of progress, e.g. a voiced remaining time or percentage completed. In such combination, the voice message may be linked to the sound origin point that changes in position to reflect the procedure progress, but this is not necessarily the case; e.g. the voice message may be delivered from a fixed point in percentual space, whereas a different audio stream can change in its virtual point of origin to represent the progress through the procedure. It is also noted that a soft or vague progress impression by encoding the progress in the spatial location of a (virtual) sound source may be more preferred than explicit status updates, e.g. as a voice message of numeric percentage completed or time to completion, for specific types of patient (e.g. children, elderly, psychiatric or neuro patients, ...) and/or in specific cases (e.g. in which active thought processes or a more analytical attention to explicit progress updates might need to be avoided, e.g. because such mental activity could interfere with a specific imaging paradigm, e.g. in fMRI).

In a second aspect, the present invention relates to a device for providing audio to a subject while undergoing a diagnostic imaging or medical procedure.

For example, a device 10 in accordance with embodiments of the present invention is schematically shown in FIG 3, as already discussed hereinabove. The device, in operation, supplies audio to a subject 200 while undergoing a diagnostic imaging or medical procedure, e.g. as depicted in strictly 2.

The device comprises an input 16 for receiving at least one audio stream. The input 16 may comprise an intercom microphone 161 (or suitable connector therefor, e.g. a microphone cable socket) for transducing (e.g. recording) the voice of an operator into the audio stream such that the operator can provide instructions and/or information to the subject by voice. The input may comprise an entertainment audio source 163 (or suitable connector therefor, e.g. via a network connection and/or cable socket) for reading or receiving a music track, an audio track associated with a video, a soundscape file and/or an audio file as said audio stream. The input may comprise an attendant microphone 162 (or suitable connector therefor, e.g. a cable socket and/or network connection) for transducing the voice of an attendant of the subject into the audio stream to comfort and/or support the subject during the procedure.

The device comprises a command center interface 17 for interactively receiving a definition of at least one source point in a three-dimensional space from an operator. The device may comprise a computer or similar processing device, e.g. comprising software and general-purpose computing hardware, and/or dedicated or programmable hardware, e.g. an application-specific integrated circuit and/or field-programmable gate array device. The command center interface 17, e.g. user interface, may thus comprise a display and/or user interface devices as generally known in the art, such as a keyboard, a mouse or other pointer device, a touchscreen, a control stick, buttons and/or keys, and/or the like.

The device comprises a three-dimensional audio processor 15, e.g. a general-purpose processor (e.g. central processing unit), a signal processor, a hardware accelerated 3D processor (e.g. as integrated in a peripheral sound processor, e.g. a sound board, and/or a graphics processing unit, particularly when suitable for general-purpose spatial processing, parallel processing and/or, for example, signal filtering), and/or similar processing device, possibly in combination with software (if not entirely implemented in hardware specifically designed and/or configured for the purposes described herein). The device may comprise a memory for storing the data being manipulated, e.g. for storing and/or buffering the audio stream(s) and/or the source point(s), and/or for storing software code to be executed to perform the described functions. The memory may comprise a transient memory, e.g. a random access memory, a persistent memory, e.g. a flash memory, and/or a long-term data storage unit, e.g. a hard drive or solid state disk, without limitation thereto.

The three-dimensional audio processor 15 is adapted to transform the at least one audio stream into at least two audio signals for outputting by corresponding sound outlets, the sound outlets being in a predetermined spatial configuration with respect to the subject during the procedure. The predetermined spatial configuration may be a-priori known, e.g. fixed in code or stored in a configuration memory, may be configurable, e.g. via settings in the command center interface, and/or may be determined by a calibration routine, e.g. based on sensor data.

The device comprises an output 18 for outputting the at least two audio signals to said corresponding sound outlets to produce sound audible by the subject during said procedure. The output may thus comprise the sound outlets, e.g. at least two speakers or a headphone set, or may be adapted to connect to the sound outlets, e.g. via an audio plug socket and/or cable. Thus, the device (e.g. the output 18) may comprise headphones 204 that comprise at least two sound outlets for emitting sound to the respective ears of the subject 200.

The device may also comprise a video display 206 configured to be viewed by the subject 200 during the procedure.

The three-dimensional audio processor is adapted to spatially encode the audio stream into the at least two audio signals such that the produced sound corresponding to the or each audio stream appears, to the subject, to originate from a corresponding defined source point or source points, e.g. as defined by an operator in the command center interface.

The device may comprise a further input 19 for receiving position and/or orientation information from a diagnostic imaging or medical system adapted to perform said diagnostic imaging or medical procedure. For example, to receive the position and/or orientation information from a magnetic resonance imaging scanner or computed tomography scanner (without limitation thereto). The position and/or orientation information is indicative of a position and/or orientation of the subject and/or of the subject's head, and/or of the sound outlets in a fixed arrangement with respect to the subject (or subject's head).

The three-dimensional audio processor 15 may be adapted to take the position and/or orientation information into account, such that a change in the position and/or orientation is compensated for, e.g. by adjusting the source point or reference position information of the outlet(s). Thus, a virtual sound space may be simulated by the three-dimensional audio processor 15 that is independent of changes of the spatial configuration of the sound outlets and/or position and/or orientation of the subject during the procedure, e.g. movement of the subject may be reflected by corresponding movement in the virtual sound environment being simulated to enhance the sense of consistency of the experience.

In a third aspect, the present invention relates to a diagnostic imaging system. The system comprises a diagnostic imaging scanner 201 for acquiring diagnostic images of a subject 200 while said subject is positioned inside an examination zone of the scanner. For example, the scanner may comprise a magnetic resonance imaging scanner and/or a computed tomography scanner, without limitation thereto. The system further comprises a device 10 in accordance with embodiments of the second aspect of the present invention.

A diagnostic imaging system in accordance with embodiments of the present invention may also comprise an automated patient couch 203 for (physically) supporting the subject 200 in the examination zone during the image acquisition, i.e. during the imaging procedure, in which position and/or orientation information of the automated patient couch is provided via said further input 19 to the device 10, e.g. by a connection between a controller and/or actuator of the couch and the further input 19. Worded differently, the further input 19 may comprise the automated patient couch.

In a fourth aspect, the present invention relates to a computer-program product for performing a method in accordance with embodiments of the first aspect of the present invention when executed by a computer, processor or suitable (e.g. programmable) processing device.

Other features, or details of the features described hereinabove, of a device, system and/or computer-program product in accordance with embodiments of the present invention shall be clear in view of the description provided hereinabove relating to a method in accordance with embodiments of the present invention. In a further aspect, the present invention also relates to a medical system for performing a medical procedure, e.g. a therapeutical invention, such as a radiotherapy system (without limitation thereto). Features of such system may essentially correspond to features of the medical imaging system as already discussed hereinabove, mutatis mutandis.

## Claims

1. A method (100) for providing audio to a subject (200) undergoing a diagnostic imaging or medical procedure, the method comprising:
- receiving (101) at least one audio stream;
- receiving (102) a definition of at least one source point in a three-dimensional space;
- transforming (103) the at least one audio stream into at least two audio signals for corresponding sound outlets, the sound outlets being in a predetermined physical spatial configuration with respect to the subject during the procedure, and
- outputting (105) the at least two audio signals to the corresponding sound outlets to produce sound audible by the subject,
- wherein said transforming (103) comprises spatially encoding the audio stream into the at least two audio signals such that the produced sound corresponding to the audio stream appears, to the subject, to originate from the defined source point.

2. The method of claim 1, wherein said transforming (103) comprises applying different filters to the audio stream to obtain the corresponding at least two audio signals, in which each of said filters is constructed by taking a head-related transfer function into account that represents a response needed at the corresponding sound outlet to emulate sound emitted from the source point.

3. The method of claim 2, comprising a calibration before performing the procedure, in which a response recorded by a microphone, which is inserted in the ear canal of the subject; to an emitted test sound is used to calibrate said head-related transfer function.

4. The method of any of the previous claims, comprising receiving (104) position and/or orientation information from a diagnostic imaging or medical system adapted to perform said imaging or medical procedure,
wherein said position and/or orientation information is indicative of a position and/or orientation of the subject and/or the subject's head,
wherein said step of transforming (103) the at least one audio stream comprises taking said position and/or orientation information into account, such that a change in said position and/or orientation is compensated for,
and such that a virtual sound space is simulated by the transformation (103) that is independent of changes of the spatial configuration of the sound outlets and/or position and/or orientation of the subject during the procedure.

5. The method of claim 4, wherein said receiving (104) of position and/or orientation information comprises receiving a position and/or orientation from a controller of an automated patient couch that supports the subject during the procedure, and/or
wherein said receiving (104) of position and/or orientation information comprises receiving pose information indicative of a pose of the subject during the procedure and/or
wherein said receiving (104) of position and/or orientation information comprises receiving sensor data, in which the subject is monitored during the procedure by acquiring said sensor data and wherein said sensor data is furthermore analyzed to determine a position and/or orientation of the subject and/or the subject's head.

6. The method of any of the previous claims, wherein receiving (102) said definition of one or more points comprises presenting a graphical user interface to an operator in which the one or more points are represented graphically, wherein said graphical user interface allows the graphical representation of the at least one source point to be manipulated by the operator so as to define and/or change said at least one source point.

7. The method of any of the previous claims, comprising outputting (106) a two-dimensional or three-dimensional video stream to at least one display to show visual content to the subject to form a virtual or augmented reality simulation in combination with the at least two audio signals.

8. The method of any of the previous claims, wherein said at least one source point is shifted in the three-dimensional space during the procedure in accordance with a progress of the procedure to represent said progress by perceptual movement of the virtual source of the audio stream for the subject.

9. A device (10) for providing audio to a subject (200) while undergoing a diagnostic imaging or medical procedure, the device comprising:
- an input (16) for receiving at least one audio stream;
- a command center interface (17) for interactively receiving a definition of at least one source point in a three-dimensional space from an operator;
- a three-dimensional audio processor (15) for transforming the at least one audio stream into at least two audio signals for outputting by corresponding sound outlets, the sound outlets being in a predetermined spatial configuration with respect to the subject during the procedure; and
- an output (18) for outputting the at least two audio signals to said corresponding sound outlets to produce sound audible by the subject during said procedure,
wherein the three-dimensional audio processor is adapted to spatially encode the audio stream into the at least two audio signals such that the produced sound corresponding to the or each audio stream appears, to the subject, to originate from its corresponding defined source point or source points.

10. The device of claim 9, comprising a further input (19) for receiving position and/or orientation information from a diagnostic imaging or medical system adapted to perform said diagnostic imaging or medical procedure,
wherein said position and/or orientation information is indicative of a position and/or orientation of the subject and/or of the subject's head,
wherein said three-dimensional audio processor (15) is adapted to take said position and/or orientation information into account, such that a change in said position and/or orientation is compensated for,
and such that a virtual sound space is simulated by the three-dimensional audio processor (15) that is independent of changes of the spatial configuration of the sound outlets and/or position and/or orientation of the subject during the procedure.

11. The device of claim 9 or claim 10, wherein said input (16) comprises an intercom microphone (161) for transducing the voice of an operator into the audio stream such that the operator can provide instructions and/or information to the subject by voice, and/or
wherein said input (16) comprises an entertainment audio source (163) for reading or receiving a music track, an audio track associated with a video, a soundscape file and/or an audio file as said audio stream, and/or
wherein said input (16) comprises an attendant microphone (162) for transducing the voice of an attendant of the subject into the audio stream to comfort and/or support the subject during the procedure.

12. The device of any of the claims 9 to 11, comprising headphones (204) that comprise at least two sound outlets for emitting sound to the respective ears of the subject (200), and/or comprising a video display (206) configured to be viewed by the subject (200) during the procedure.

13. A diagnostic imaging system, the system comprising:
- a diagnostic imaging scanner (201) for acquiring diagnostic images of a subject (200) while said subject is positioned inside an examination zone of the scanner, and
- a device (10) in accordance with any of the claims 9 to 12.

14. The diagnostic imaging system of claim 13, comprising an automated patient couch (203) for supporting the subject (200) in the examination zone during the image acquisition, wherein position and/or orientation information of the automated patient couch is provided via said further input (19) to the device (10).

15. A computer-program product for performing a method in accordance with any of the claims 1 to 8 when executed by a computer.
